# EUROPÄISCHE PATENTANMELDUNG

(11) **EP 4 796 624 A1**
(43) Veröffentlichungstag der Anmeldung: **26.08.2026**
(21) Anmeldenummer: 26158780.2
(22) Anmeldetag: 16.02.2026
(51) Int. Cl.: C12M 1/107, C12M 1/00

(54) **VERFAHREN ZUR AUFBEREITUNG VON BIOGAS**

(30) Priorität: 19.02.2025 DE 102025106292
(71) Anmelder: Zweckverband Klärwerk Steinhäule (ZVK) (Körperschaft des öffentlichen Rechts), 89233 Neu-Ulm (DE)
(72) Erfinder: Fuchs, Jonathan, 89233 Neu-Ulm (DE); Herrmann, Volker, 89233 Neu-Ulm (DE)
(74) Vertreter: Epping - Hermann - Fischer

(57) **Zusammenfassung**

Die Erfindung betrifft ein Verfahren zur gleichzeitigen Aufbereitung eines Rohgases (5) und eines Fermentationsmediums (4), aufweisend die Schritte:
- Verminderung der im Fermentationsmedium (4) enthaltenen Gase, insbesondere Methan, und gleichzeitige Bereitstellung eines beladenen Gases,
- Bereitstellung eines gereinigten Gases (6,7) durch zumindest teilweise Abtrennung unerwünschter Gasbestandteile vom beladenen Gas zur Erhöhung des Methananteils.

## Beschreibung

Die vorliegende Erfindung betrifft ein Verfahren zur Aufbereitung von Biogas und eines Fermentationsmediums.

Biogas wird durch den Fermentierungsprozess organischer Materialien wie beispielsweise Biomasse, Gülle oder Lebensmittelabfälle gewonnen. In einer anaeroben Umgebung zersetzen Mikroorganismen diese Stoffe und erzeugen dabei ein energiereiches Gasgemisch, das hauptsächlich aus Methan und Kohlendioxid besteht. Dieses Biogas kann zur Strom- und Wärmeerzeugung genutzt oder aufbereitet als Erdgasersatz verwendet werden.

Trotz seiner Vorteile, wie der Nutzung nachwachsender Rohstoffe und der Reduktion von Treibhausgasemissionen, ist die Nutzung solchen Biogases aktuell auch mit Nachteilen verbunden.

Dazu gehören die begrenzte Reinheit von aus Fermentierung gewonnenen Gasen, ein niedriger Heiz- oder Brennwert und potenzielle Umweltprobleme durch unerwünschte Gasbestandteile oder, im Falle einer Aufbereitung des Biogases zu Biomethan, aufwändige Aufbereitungsverfahren.

Die vorliegende Erfindung betrifft ein Verfahren zur Aufbereitung eines Rohgases und eines Fermentationsmediums gemäß den Ansprüchen.

Insbesondere betrifft die vorliegende Erfindung ein Verfahren zur gleichzeitigen Aufbereitung eines Rohgases und eines Fermentationsmediums.

Ein Fermentationsmedium ist hierbei jedes Medium, das zumindest teilweise einer Fermentierung unterworfen war, egal ob z.B. in einem technischen Prozess oder durch Umwelteinflüsse.

Insbesondere zielt die Erfindung auf ein Fermentationsmedium, das durch die zumindest teilweise Fermentierung von Klärschlamm gewonnen ist. Das Fermentationsmedium kann jedoch auch durch die zumindest teilweise Fermentierung jedes geeigneten, insbesondere biologischen, ersten Mediums gewonnen sein. Das erste Medium kann zum Beispiel Grüngut, Getreide, Gärreste, Bioabfall, Kompost, tierische Rest- und Abfallstoffe, Wirtschaftsdünger, Klärschlamm, Milch oder Fette andere teilweise vergärbare bzw. fermentierbare Substanzen oder Ähnliches umfassen.

Das Fermentationsmedium umfasst vorwiegend, aber nicht ausschließlich, Feststoffe und Flüssigkeit. Insbesondere kann es sich bei dem Fermentationsmedium um einen Schlamm handeln, der im Wesentlichen Wasser und ca. 0,1-10 %, insbesondere 2-3 % (Massenprozent), feste Bestandteile enthält. Weiterhin umfasst das Fermentationsmedium Gase, insbesondere Gase, die während der Fermentation entstanden sind.

Unter Fermentation wird hier insbesondere die vorwiegend anaerobe Vergärung bzw. Verstoffwechselung organischen Materials durch Mikroorganismen verstanden. Auch die Erzeugung von Synthesegas z.B. in thermischen Prozessen kann gemeint sein.

Das Rohgas im Sinne der Erfindung ist z.B. ein Biogas, Deponiegas, Grubengas, Schwachgas, Erdgas oder Mischungen daraus, also ein erdgasähnliches Gasgemisch, das bevorzugt insbesondere durch die anaerobe Zersetzung, insbesondere Fermentation, von Biomasse gebildet wurde.

Das Rohgas weist insbesondere einen hohen Methananteil von bevorzugt über 50 % (in Bezug auf das Gas immer Volumenprozent bezogen auf Normkubikmeter) und einen Anteil an Kohlenstoffdioxid von insbesondere 25 - 50 % auf.

Weiterhin können Schwefelwasserstoff, Ammoniak, Stickstoff, Wasserstoff, Sauerstoff und andere Gasen enthalten sein.

Das Fermentationsmedium und das Rohgas können insbesondere durch denselben Fermentationsprozess gebildet worden sein.

Der Fermentationsprozess umfasst insbesondere auch mehrstufige Vergärungen, wobei dass Fermentationsmedium und das Rohgas jeweils aus den gleichen oder unterschiedlichen Stufen der Fermentation stammen können. Besonders bevorzugt entstehen Rohgas und Fermentationsmedium in einem einstufigen Prozess.

Das erfindungsgemäße Verfahren weist zumindest die folgenden Schritte auf:
In einem Schritt werden das Rohgas und das Fermentationsmedium bevorzugt lokal getrennt bereitgestellt.

Das Fermentationsmedium und das Rohgas können, müssen aber nicht aus derselben Quelle stammen.

In einem Schritt werden die im Fermentationsmedium enthaltenen Gase, insbesondere das im Fermentationsmedium enthaltene Methan, aber z.B. auch Kohlenstoffdioxid und Ammoniak, das gemäß Ammonium-Ammoniak-Gleichgewicht als Ammoniak oder Ammonium vorliegen kann, vermindert. Dies erfolgt durch zumindest teilweise Desorption der im Fermentationsmedium enthaltenen Gase, insbesondere des dort enthaltenen Methans, und durch Aufnahme (z.B. Absorption) dieser Gase im Rohgas (Gas-Stripping).

Insbesondere kann das Ammonium-Ammoniak-Gleichgewicht durch die Anpassung der Temperatur, des Drucks und des pH-Werts im Sinne der Erfindung manipuliert, also in Richtung des Ammoniaks verschoben werden, was in den einzelnen Ausführungsformen beschrieben ist. Umso weiter das Gleichgewicht verschoben wird, desto schneller läuft der Prozess ab.

Gleichzeitig wird ein beladenes Gas bereitgestellt bzw. erzeugt, wobei zumindest die Menge an Methan im Rohgas durch Absorption aus dem Fermentationsmedium vorteilhaft gesteigert wird. Somit steigt beispielsweise vorteilhaft auch der Energiegehalt und Brennwert bzw. Heizwert des beladenen Gases gegenüber dem Rohgas.

Das gleichzeitige Desorbieren von Gasbestandteilen aus dem Fermentationsmedium durch das Absorbieren dieser Gasbestandteile im Rohgas wird als Strippen oder Stripping bezeichnet.

Das Strippen findet in einem ersten Reaktor statt, der auch als Stripper bezeichnet werden kann. Der erste Reaktor kann insbesondere als Kolonne, als Riesler, als Rührkessel, als Rohrstrecke, o.ä. ausgeführt sein.

Um die Desorption der Gasbestandteile aus dem Fermentationsmedium zu unterstützen und das Desorptions-Absorptions-Gleichgewicht, bzw. insbesondere auch das Ammonium-Ammoniak-Gleichgewicht, in Richtung eines vermehrten Gasüberganges in das Rohgas zu verschieben, wird das Strippen bevorzugt bei 0 bis 2 bar absolut (bara), besonders bevorzugt bei Unterdruck bei unter 1 bar absolut durchgeführt.

Weiterhin wird die Temperatur im Stripper zu demselben Zweck bevorzugt bei mindestens Raumtemperatur, bevorzugter 30 °C, bevorzugter 50 °C, bevorzugter bei 50 bis 95 °C, bevorzugter bei 55 bis 75 °C eingestellt.

Optional können während des Strippens auch weitere Fremdgase neben dem Rohgas bzw. gereinigten Gas als Stripp-Gas eingesetzt werden.

In einem weiteren Schritt wird ein gereinigtes Gas bereitgestellt, indem das beladene Gas, oder das beladene Gas und das Rohgas durch eine zumindest teilweise Abtrennung unerwünschter Gasbestandteile, darunter zumindest eines von Ammoniak, Kohlenstoffdioxid und Schwefelwasserstoff gereinigt wird.

Unter dem Reinigen wird also insbesondere das Abtrennen von nicht-kohlenwasserstoffhaltigen Gasbestandteilen aus dem Rohgas oder beladenen Gas verstanden.

Vorteilhaft wird zumindest der Methananteil im beladenen Gas somit im Vergleich zum Vorzustand des Gases erhöht.

Ein weiterer Vorteil des Verfahrens ist, dass auch anderweitige Störstoffe wie VOC (Volatile Organic Compounds) oder Siloxane während des beschriebenen Prozesses entfernt werden können, die für eine anschließende Nutzung des Gases störend sein können.

Die beschriebene Gasreinigung findet in einem zweiten Reaktor, insbesondere einem Gaswäscher oder Trockenabsorber statt.

Die Gasreinigung findet optional unter Verwendung von Wasser, neutralen, sauren oder basisch wirkenden Bestandteilen, Aufschlämmungen oder Eluaten statt, bevorzugt aber zumindest unter Verwendung von Wasser. Alternativ genügt auch eine Kühlung im zweiten Reaktor, um Feuchtigkeit aus dem Gas zu kondensieren und damit Gaswäsche zu betreiben.

Die Gasreinigung ist insbesondere eine Gaswäsche oder eine Trockenabsorption, in dem Sinn, dass Gasbestandteile unter Zuhilfenahme von einem Waschmedium, insbesondere einer Waschflüssigkeit oder eines Waschschlammes (flüssig-fest), oder unter Hilfe eines Trockenabsorptionsmittels bevorzugt bestehend aus Rest- und Abfallstoffen aus dem Gas entfernt, insbesondere aus dem Gas heraus von der Waschflüssigkeit oder dem Waschschlamm oder dem Trockenabsorptionsmittel absorbiert werden.

Das Waschmedium kann z.B. auch Branntkalk, Salzlösungen, Salze, o.ä. enthalten. Wird im Verfahren in einem der Gasreinigungsschritte mit sauer oder basisch wirkendenden Substanzen gearbeitet, so sind diese bevorzugt Rest- und Abfallstoffe aus anderen Prozessen bzw. (Zwischen- oder Neben-)Produkte daraus.

Das Strippen des Fermentationsmediums im Rohgas bzw. (wieder) gereinigten Gas und die Gasreinigung des Rohgases oder (wieder) beladenen Gases finden bevorzugt mehrmals hintereinander statt, um ein möglichst entgastes Fermentationsmedium und ein möglichst methanreiches gereinigtes Gas, das möglichst frei von ungewollten Gasbestandteilen ist, zu erhalten.

Insbesondere kann das Rohgas zur Aufbereitung auch mehrere zweite Reaktoren bzw. Gaswäscher durchströmen.

Gemäß einer Ausführungsform werden das Rohgas und das Fermentationsmedium durch Fermentation in einem gemeinsamen, bevorzugt einem einzelnen Fermenter gewonnen bzw. erzeugt.

Das Rohgas und das Fermentationsmedium werden bereitgestellt, indem diese bevorzugt (örtlich) getrennt aus dem Fermenter entnommen werden.

Das Fermentationsmedium kann zur Bereitstellung weiterhin optional vor, im oder nach dem Fermenter erhitzt, desintegriert oder durch Zugabe von Fällmitteln aufbereitet werden, um biologische Strukturen zu zerstören und im Verfahren ungewollte Stoffe durch Fällung als unlöslicher Feststoff im Fermentationsmedium zu fixieren.

Gemäß einer Ausführungsform wird das gereinigte Gas zumindest teilweise in den Fermenter zurückgeführt.

So kann der Anteil ungewollter Substanzen wie Ammoniak, das als Zellgift wirken kann, im Fermenter vorteilhaft verringert werden.

Der Methananteil im Gas kann so weiter gesteigert werden.

Gemäß einer Ausführungsform weist das Verfahren weitere Schritte auf.

Ein optionaler Verfahrensschritt umfasst die zumindest teilweise Rückführung des gereinigten Gases in den ersten Reaktor und erneutes Strippen zur Bereitstellung eines beladenen Gases (siehe auch oben).

Ein weiterer optionaler Verfahrensschritt umfasst die erneute Abtrennung der unerwünschten Gasbestandteile des beladenen Gases zur Bereitstellung eines gereinigten Gases mittels des mindestens einen zweiten Reaktors (siehe auch oben).

Gemäß einer Ausführungsform werden bei der Verminderung der im Fermentationsmedium enthaltenen Gase (Strippen) neben Methan, gleichzeitig auch zumindest Ammoniak und Kohlenstoffdioxid aus dem Fermentationsmedium entzogen.

Bei der anschließenden Abtrennung unerwünschter Gasbestandteile aus dem beladenen Gas (Gaswäsche) werden dann vorteilhaft direkt entsprechende zumindest aus Ammoniak und Kohlenstoffdioxid gebildete Salze wie Ammoniumsalze und Carbonate ausgefällt oder als eine wässrige Salzlösung entnommen, wie zum Beispiel Ammoniumcarbonat, - hydrogencarbonat oder -carbamat.

Um übriges Ammoniak oder Kohlenstoffdioxid abzutrennen ist dann insbesondere bevorzugt eine Gaswäsche im entsprechend gegenteiligen sauren oder basischen Milieu durchzuführen.

Gemäß weiteren Ausführungsformen werden diese Salze in anschließenden Prozessen wieder, z.B. thermisch oder elektrochemisch, gespalten, um Ammoniak und/oder Kohlenstoffdioxid bereitzustellen.

Alternativ können die Salze anschließend chemisch mittels einer sauer wirkenden Substanz gespalten werden, um ein weiteres ammoniakhaltige Salz und Kohlenstoffdioxid in Gasform zu erhalten.

Alternativ können die Salze anschließend chemisch mittels einer basisch wirkenden Substanz gespalten werden, um ein weiteres Kohlendioxid enthaltendes Salz und Ammoniak in Gasform zu erhalten.

Gemäß einer Ausführungsform wird das Fermentationsmedium nach der Verminderung der darin enthaltenen Gase anschließend einer Fest-Flüssig-Phasentrennung (Fest-Flüssig-Trennung) unterzogen.

Insbesondere, wenn das Fermentationsmedium ein Schlamm, insbesondere ein Klärschlamm aus einem Klärprozess, ist, werden dann feste und flüssige Bestandteile, insbesondere Prozessflüssigkeit aus dem Klärprozess voneinander getrennt und die festen Bestandteile bevorzugt zur weiteren energetischen Verwertung getrocknet.

Hierfür ist die zuvor erfolgte Entgasung insbesondere von Vorteil, um die ungewollte Freisetzung potenziell umweltgesundheits- und maschinenschädlicher sowie korrosiver Gase zu vermeiden.

Durch die zusätzliche Ausfällung übrigen Ammoniaks und, oder Phosphats durch die Zugabe von Chemikalien, wie Fällmitteln, kann die Fest-Flüssig Trennung weiter verbessert werden.

Gemäß einer Ausführungsform wird das Fermentationsmedium kontinuierlich zwischen dem Fermenter und dem Stripper ausgetauscht, wobei im Stripper zumindest eines von Ammonium in Form von Ammoniak, Kohlenstoffdioxid, Schwefelwasserstoff oder Methan aus dem Fermentationsmedium entzogen wird und wobei das Fermentationsmedium anschließend zumindest teilweise wieder in den Fermenter rückgeführt wird.

So kann der Anteil ungewollter Substanzen wie z.B. Ammoniak, das als Zellgift wirken kann, im Fermenter vorteilhaft verringert werden.

Gemäß einer Ausführungsform wird das entgaste Fermentationsmedium nach dem ersten Reaktor, und vor einer optionalen Fest-Flüssig-Trennung, in einen dritten Reaktor überführt, in welchem das Fermentationsmedium mit einem zweiten Gas so in Kontakt gebracht wird, dass Fermentations-Organismen im Fermentationsmedium inaktiviert werden, z.B. um das Fermentationsmedium oder die später von Wasser weitgehend befreiten Feststoffe lagerstabiler bereitzustellen.

Das zweite Gas kann hierzu insbesondere über Raumtemperatur erwärmt sein. Insbesondere wird das Fermentationsmedium vor dem dritten Reaktor weiter erwärmt.

Gemäß einer Ausführungsform wird als das zweite Gas insbesondere Luft, Abluft aus Klär- oder Industrieprozessen oder Abgas aus Verbrennungsprozessen verwendet, wobei in dem Gas befindliche Schadstoffe vorteilhaft zumindest teilweise durch das Fermentationsmedium absorbiert werden.

Bevorzugt wird das aus dem dritten Reaktor entnommene Gas anschließend einer weitergehenden Abluftreinigung unterzogen, beispielsweise im biologischen Klärprozess von Abwässern.

Gemäß einer Ausführungsform werden in den ersten Reaktor weiterhin Fällmittel oder sauer bzw. basisch wirkende Stoffe oder Lösungen zugegeben, um das Absorptions-/Desorptions-Gleichgewicht von im Fermentationsmedium enthaltene Gasbestandteilen während des Strippens gezielt zu verändern und im Fermentationsmedium gelöste Bestandteile, insbesondere Phosphate, gezielt auszufällen.

Insbesondere können z.B. Calciumoxid, -hydroxid, Natronlauge, Magnesiumhydroxid, oder Rest- und Abfallstoffe etc. zugegeben werden.

Dies verbessert vorteilhaft insbesondere eine spätere Fest-Flüssig-Trennung und verringert den Bedarf an weiteren Betriebsstoffen wie polymeren Flockungsmitteln.

Gemäß einer Ausführungsform wird in den ersten Reaktor weiterhin Brüdenkondensat eingeführt, das im ersten Reaktor gleichzeitig mit dem Fermentationsmedium von enthaltenen Gasen zumindest teilweise befreit wird.

Wie dem Fachmann bekannt ist, wird als Brüdenkondensat insbesondere das Kondensat bezeichnet, das beim Verdampfen von Flüssigkeiten z. B. in Trocknungsanlagen von Kläranlagen und dem nachfolgenden Kondensieren der gasförmigen Brüden entsteht. Dieses Kondensat enthält in Wasser gelöste Stoffe und muss als Abwasser weiterbehandelt werden.

Zumindest ein Teil des Brüdenkondensats wird optional durch Trocknung des von enthaltenen Gasen zumindest teilweise befreiten und teilweise von Wasser abgetrennten Fermentationsmediums und anschließende Kondensierung generiert.

Die Kondensation der z.B. aus dem Fermentationsmedium gewonnenen gasförmigen Brüden findet bevorzugt vor dem oder im ersten Reaktor statt, um Wärme zurückzugewinnen und das Fermentationsmedium vor dem oder beim Stripping zu erwärmen (siehe oben).

Gemäß einer Ausführungsform werden nach der Verminderung der im Fermentationsmedium enthaltenen Gase durch (wiederholtes) Strippen im ersten Reaktor die im Fermentationsmedium enthaltenen Gase durch Vakuumentgasung in einem zweiten Entgasungsschritt weiter vermindert oder komplett entfernt.

Gemäß einer Ausführungsform durchströmt das Rohgas und/oder das beladene Gas und/oder das gereinigte Gas in einer beliebigen Phase des Verfahrens bevorzugt jedoch nach abgeschlossener wiederholter Reinigung per Gaswäsche weiterhin einen Gasfilter, insbesondere einen Sand,- Kies- oder einen Aktivkohlefilter, zur weiteren Reinigung, insbesondere von ungewollten Substanzen.

Gemäß einer Ausführungsform wird die Wärmeenergie aus dem Verfahren rückgewonnen und bevorzugt zur Vorwärmung des Fermentationsmediums vor dem ersten Reaktor oder während des Fermentationsprozesses genutzt.

Insbesondere die Wärmeenergie des im Verfahren behandelten Gasstromes oder der Prozessflüssigkeit können z.B. durch Wärmetauscher zurückgewonnen werden und zur Erwärmung des Fermenters oder des ersten Reaktors wiedergenutzt werden.

Gemäß einer Ausführungsform wird durch die Zugabe von Calciumsulfat im zweiten Reaktor Ammoniumsulfat und Calciumcarbonat gebildet.

So werden Ammoniak und Kohlenstoffdioxid vorteilhaft aus dem Gas (gemeint ist hier im allgemeinen Fall immer das Rohgas bzw. das gereinigte Gas bzw. das beladene Gas) oder dem Fermentationsmedium entfernt und Salze gewonnen.

Gemäß einer Ausführungsform wird das gereinigte Gas einer weitergehenden Nachbehandlung unterzogen, um einer lokalen und, oder über ein Verteilnetz räumlich getrennten, energetischen und/ oder stofflichen Nutzung zugeführt zu werden.

Im Folgenden wird die Erfindung anhand von
Ausführungsbeispielen und dazugehörigen Figuren näher beschrieben.

Die Figuren zeigen schematisch sechs beispielhafte Verfahren, in denen die im Wesentlichen zuvor definierten Schritte in geeigneter Reihenfolge durchgeführt und gekoppelt werden.

Ähnliche oder augenscheinlich gleiche Prozessschritte bzw. Prozesselemente sind in den Figuren mit dem gleichen Bezugszeichen versehen.

Die Beziehungen und Schritte sind in den Figuren nur schematisch dargestellt und nicht ergiebig oder einschränkend zu interpretieren.

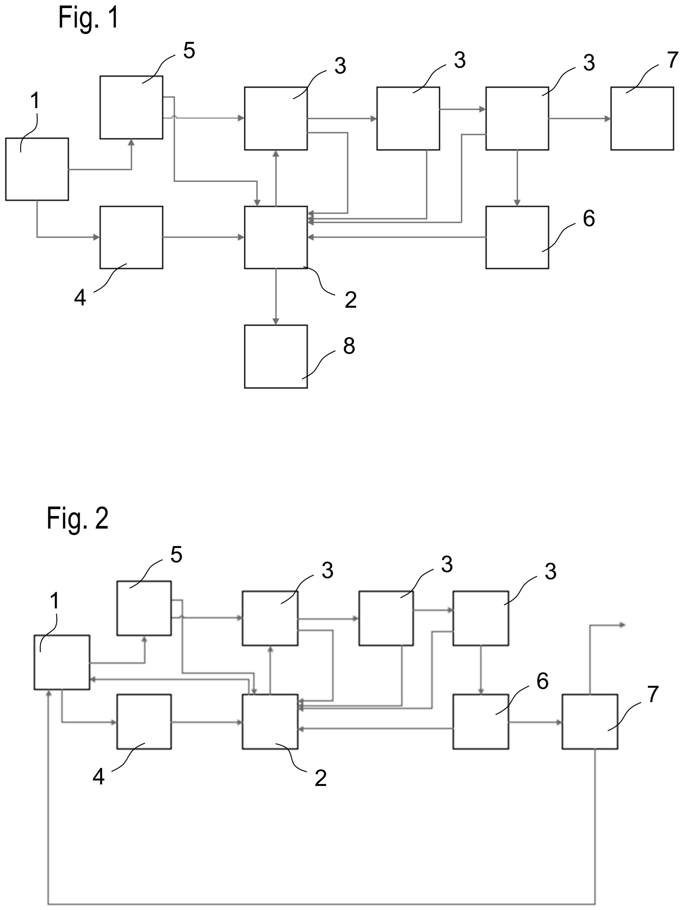

Figur 1 zeigt schematisch eine erste Ausführungsform des Verfahrens. Ein Rohgas 5 und ein Medium 4 (das Fermentationsmedium 4) werden aus einem Fermenter 1 als zwei getrennte Fraktionen entnommen. Alternativ kann das Rohgas 5 zumindest teilweise auch aus anderen Quellen stammen.

In Bezug auf den weiteren Prozess werden die zwei Fraktionen auch als Medium 4 und als Gas bezeichnet.

Anschließend wird das Medium 4 in einem ersten Reaktor 2 (dem Stripper 2) durch Strippen mit dem Gas, das als Stripp-Gas fungiert, zumindest teilweise entgast, also wie oben beschrieben werden Gasbestandteile aus dem Medium 4 in das Gas abgegeben.

Das so beladene Gas wird anschließend in einem oder mehreren zweiten Reaktoren 3 (den Gaswäschern 3), zum Beispiel den drei gezeigten Gaswäschern 3 durch Gaswäsche im wässrigen, sauren oder basischen Milieu von ungewollten Gasbestandteilen gereinigt.

Diese Reinigung findet insbesondere in Form der Gaswäsche durch Absorption der in ungewollten Gasbestandteile in zumindest teilweise flüssigen Waschsubstanzen statt (siehe oben).

Nach jedem der (z.B. drei) Gaswäscheschritte kann das so gereinigte Gas 6 wieder als Stripp-Gas zurück in den ersten Reaktor 2 geführt werden.

Das schließlich vollends in den Gaswäschern 3 aufgereinigte Gas 7 kann schließlich zur weiteren Verwertung, insbesondere auch zur weiteren Aufreinigung, aus dem Prozess entnommen werden.

Insbesondere kann z.B. immer so viel Gas 7 aus dem Prozess entnommen werden, wie neues Rohgas 5 in den Prozess eingeführt wird.

Das bevorzugt möglichst vollständig entgaste Medium 4 wird anschließend aus dem Stripper 2 entnommen und einer Fest-Flüssig-Trennung 8 unterzogen. Insbesondere handelt es sich bei dem Medium 4 um einen Klärschlamm, der anschließend an den Stripp-Schritt entwässert wird, wobei eine Prozessflüssigkeit aus dem Klärprozess aus dem Klärschlamm abgezogen wird. Der entwässerte und anschließend getrocknete Klärschlamm (Schlamm) bzw. der daraus gewonnene Feststoff kann anschließende z.B. energetisch verwertet werden.

In Figur 2 ist gezeigt, dass das Verfahren z.B. zusätzlich so modifiziert sein kann, dass das Rohgas 5 und/oder das Medium 4 kontinuierlich aus dem Fermenter 1 entnommen und wieder zurückgeführt wird, insbesondere um Ammoniumverbindungen aus dem Fermenter 1 zu entfernen und somit eine störende Ammoniumhemmung der Fermentations-Organismen im Fermenter 1 zu verringern.

In Figur 2 ist insbesondere gezeigt, dass das Medium 4 insbesondere nach dem Strippen, wobei vorteilhaft zumindest teilweise Ammoniumverbindungen aus dem Medium 4 entfernt werden und als Ammoniak in die Gasphase übergehen, zumindest teilweise in den Fermenter 1 zurückgeführt wird.

Insbesondere kann immer so viel Medium 4 zurückgeführt und/oder aus dem Prozess entnommen werden, wie neu in den Prozess zugeführt wird (kontinuierlicher Betrieb).

Weiterhin ist gezeigt, dass das aufgereinigte Gas 6 zumindest nur teilweise aus dem Prozess entnommen wird, während ein Teil des gereinigten Gases 6 in den Fermenter 1 zurückgeführt wird. Die während dem Strippen in das Gas aufgenommen Ammoniumverbindungen sind dann vorteilhaft zuvor während der Gaswäsche aus dem Gas entfernt worden.

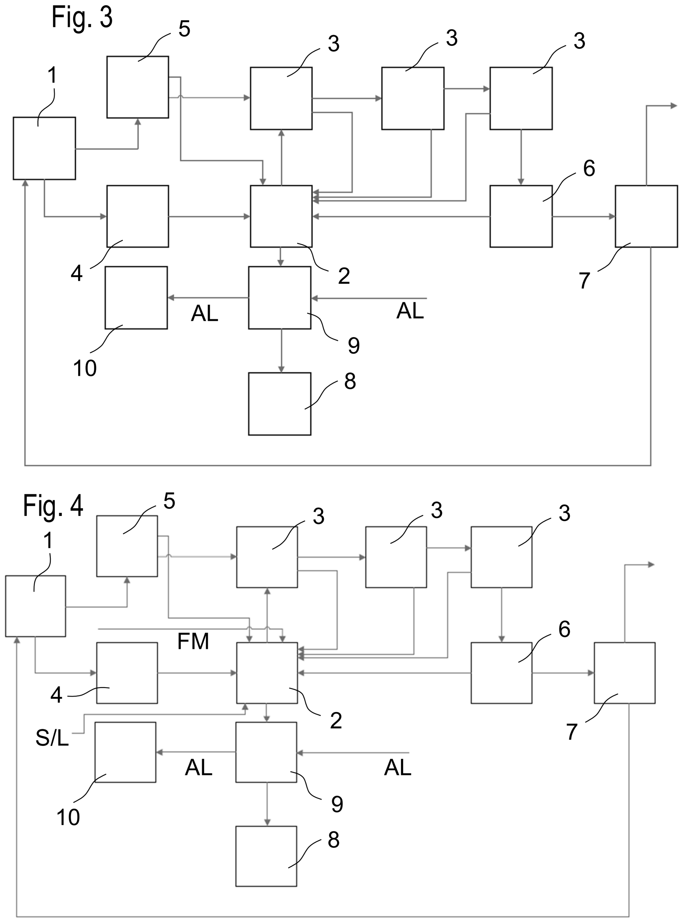

In Figur 3 ist erneut im Wesentlichen das Verfahren als Figur 1 gezeigt.

Weiterhin sind nachgelagerte Schritte zur Inaktivierung des Mediums 4 nach der Entgasung im Stripper 2 gezeigt.

Dazu wird das zumindest teilweise entgaste Medium 4 aus dem Stripper 2 in einen dritten Reaktor 9, den Inaktivierter 9, überführt und dort z.B. mit Luft, Abgas, Abluft etc. (im Folgenden Abluft), die oder das zumindest teilweise Sauerstoff enthält, inaktiviert, sodass verbleibende fermentierte Organismen bevorzugt inaktiviert werden.

Gleichzeitig wird die Abluft dabei vorteilhafterweise durch Absorption von schädliche Substanzen in das Fermentationsmedium 4 aufgereinigt. Die Abluft kann anschließend weitergehend behandelt werden.

Nach dem Inaktivieren wird das Medium 4 weiter behandelt, insbesondere in der Fest-Flüssig-Trennung 8.

Weiterhin ist auch in Figur 3 wiederum gezeigt, dass das aufgereinigte Gas teilweise in den Fermenter 1 rückgeführt werden kann, der zum Beispiel als Gaseinpressung oder Schlaufenreaktor ausgeführt ist. Auch der kontinuierliche Austausch zwischen dem Medium 4 aus dem Stripper 2 und dem Fermenter 1 (hier nicht gezeigt) ist weiterhin möglich.

In Figur 4 ist weiterhin gezeigt, dass bei Bedarf Fällmittel in den Stripper 2 hinzugesetzt werden können, beispielsweise Fällmittel zur Phosphatausfällung.

Weiterhin können z.B. sauer oder basisch wirkende Substanzen zum selektiven Strippen entsprechend umgekehrt basischer oder saurer Gasbestandteile aus dem Medium 4 in den Stripper 2 zugesetzt werden.

Als sauer oder basisch wirkende Substanzen werden z.B. bevorzugt Abfallstoffe oder deren Eluat verwendet, da diese kostengünstig und ressourcenschonend bereitgestellt werden können, um einer Kreislaufwirtschaft zu dienen.

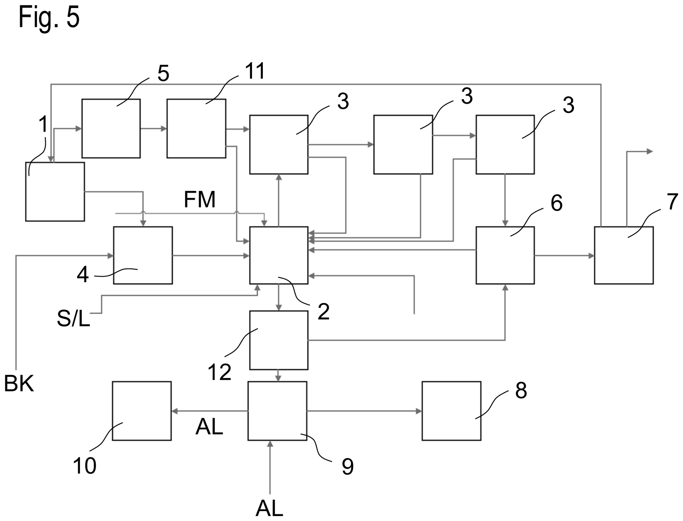

In dem in Figur 5 gezeigten Verfahren wird das Rohgas 5 zusätzlich zu bereits gezeigten Verfahrensschritten vor dem Einführen in den Stripper 2 durch einen Gasfilter 11, der zum Beispiel Sand, Kies oder Aktivkohle enthält, vorgereinigt.

Ein solcher Gasfilter 11 kann auch nach den Gaswäschern 3 zur Endreinigung des Gases vorgesehen sein.

So kann die Effektivität des Stripp-Vorgangs verbessert werden.

In den Stripper 2 können wie zuvor beschrieben weiterhin Fällmittel und Säuren oder Laugen zugesetzt werden.

Parallel können, wie schon oben beschrieben, Brüdenkondensat oder gasförmige Brüden in den Stripper 2 eingeführt werden, die dann parallel mit dem Medium 4 entgast werden.

Die Brüden werden vor oder im Stripper 2 kondensiert, wobei die abgegebene Wärme bevorzugt für die Erwärmung des Strippers 2 genutzt wird, um die Entgasung zu erleichtern.

Weiterhin kann nach dem Stripper 2 und vor einer Inaktivierung oder Fest-Flüssig-Trennung 8 des Mediums 4 ein zusätzlicher Schritt der Vakuumentgasung 12 durchgeführt werden, um eine möglichst vollständige Entgasung des Fermentationsmediums 4 zu ermöglichen und Methanschlupf weitestgehend zu minimieren.

Das im Vakuum abgeführte Gas kann anschließend mit der übrigen Gasfraktion gemischt und abgezogen werden.

Das aus dem Prozess abgezogene Gas wird bevorzugt weiter aufbereitet, zum Beispiel sauer und/oder basisch reduziert.

Das abgezogene Gas kann z.B. direkt zur thermischen Verwertung verwendet werden oder in ein (Erd-)Gasnetz eingespeist werden.

Alle einzelnen, zuvor eingeführten Prozessschritte können beliebig geeignet kombiniert werden.

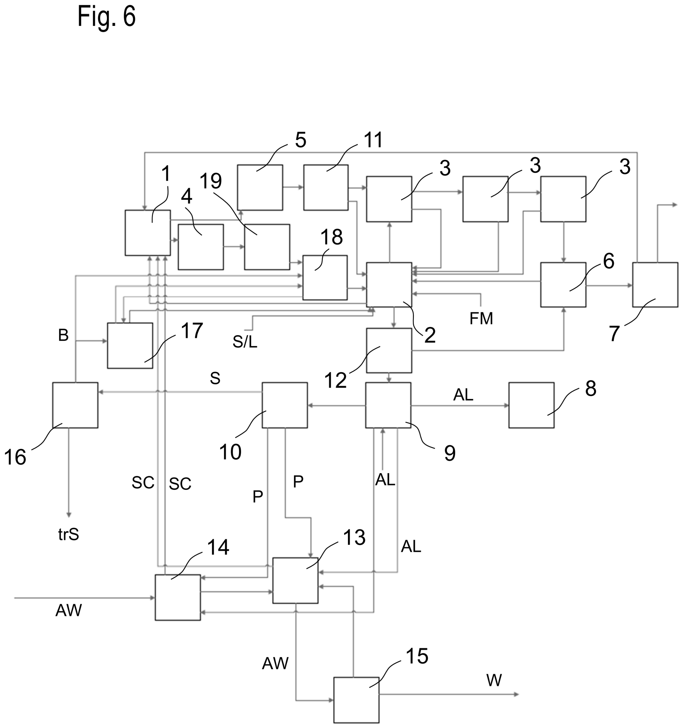

In Figur 6 ist schließlich eine Gesamtübersicht über die verschiedenen kombinierten Verfahrensschritte gezeigt.

Zusätzliche sind weitere Vor- und Nachbehandlungsschritte des Mediums 4 gezeigt.

Insbesondere ist gezeigt, wie das Fermentationsmedium 4 nach der Fest-Flüssig-Trennung 8 weiter behandelt werden kann.

Der feste Bestandteil, also der entwässerte Schlamm (Gärreste) wird bevorzugt getrocknet (16) zur Entsorgung oder weiteren Verwertung (z.B. energetische Verbrennung).

Das bei der Trocknung abgesonderte Fluid wird als Brüden bezeichnet. die Brüden werden bevorzugt in einem Brüden-Kondensator 17 kondensiert und anschließend zur weiteren Aufreinigung in den Stripper 2 eingeführt, wie oben beschrieben.

Die abgegebene Wärme, z.B. Kondensationswärme, aus dem Brüden-Kondensator 17 wird bevorzugt durch einen Wärmetauscher 18 an den Stripper 2 und, oder das Fermentationsmedium 4 abgegeben. Alternativ können die Brüden direkt im Stripper 2 kondensiert werden. Wiederum alternativ können die Brüden vor der Zugabe in den Brüdern-Kondensator bereits mittels eines Wärmetauschers 18 Wärme an das Fermentationsmedium 4 bzw. den Fermenter 1 abgeben (überhitztes Gas).

Die Zugabe der Brüden in den Stripper 2 ist optional.

Weiterhin wird der flüssige Anteil nach der Fest-Flüssig-Trennung 8 des Mediums 4 über die herkömmliche mechanische, biologische und chemische Abwasserreinigung 14, 13 weitergehend aufgereinigt. Das gereinigte Wasser kann in die Umgebung abgeführt werden oder beispielsweise über eine Viertbehandlung wie das Ulmer Verfahren mittels Pulveraktivkohle, die nach der Beladung in die biologisch/chemische Abwasserreinigung zurückgeführt wird, weiter aufgereinigt werden. Die mechanische Abwasserreinigung 14 findet, wenn nötig, in der Regel vor der biologischen/chemischen Abwasserreinigung 13 statt.

Die in der mechanischen, biologischen und chemischen Abwasserreinigung 14, 13 abgesonderten Bestandteile, insbesondere der Klärschlamm, werden im Fermenter 1 fermentiert und anschließend gemäß dem zuvor beschriebenen Prozess weiter aufbereitet.

Desgleichen kann auch die zuvor im beschriebenen Inaktivierer 9 abgezogene Abluft in das Abwasser eingeführt werden und durch die biologische und chemische Abwasserreinigung 13 weiter aufgereinigt werden.

Weitere optionale Schritte sind die zusätzliche Zerkleinerung oder mechanische Säuberung durch einen Störstoffausscheider 19 des aus dem Fermenter 1 gewonnen Mediums 4 bereits vor der Zuführung des Mediums 4 in den Stripper 2 oder einen optionalen Wärmetauscher 18.

### Bezugszeichen

- 1: Fermenter 1
- 2: erster Reaktor
- 3: zweiter Reaktor
- 4: Fermentationsmedium 4
- 5: Rohgas 5
- 6: gereinigtes Gas
- 7: Gas zur Entnahme aus dem Prozess (oder Wiederrückführung in den Fermenter 1)
- 8: Fest-Flüssig-Trennung 8
- 9: dritter Reaktor
- 10: Abluftbehandlung
- 11: Gasfilter
- 12: Vakuumentgasung
- 13: biologische/chemische Abwasserreinigung
- 14: mechanische Abwasserreinigung
- 15: weitergehende Abwasserbehandlung
- 16: Trocknung von Schlamm
- 17: Brüden-Kondensator
- 18: Wärmetauscher
- 19: Zerkleinerer
- AL: Abluftstrom
- FM: Fällmittel
- S/L: Säure oder Lauge
- BK: Brüdenkondensat
- AW: Abwasser
- SC: Schlamm aus Abwasserreinigung
- W: gereinigtes Wasser
- B: Brüden
- S: Schlamm
- trS: getrockneter Schlamm
- P: Prozesswasser

## Patentansprüche

1. Verfahren zur gleichzeitigen Aufbereitung eines Rohgases (5) und eines Fermentationsmediums (4), aufweisend die Schritte:
- Bereitstellung des Rohgases (5) und des Fermentationsmediums (4),
- Verminderung der im Fermentationsmedium (4) enthaltenen Gase, insbesondere Methan, und gleichzeitige Bereitstellung eines beladenen Gases durch Steigerung zumindest der Menge an Methan im Rohgas (5) durch Strippen des Fermentationsmediums (4) mit zumindest einem Teil des Rohgases (5) in einem ersten Reaktor (2),
- Bereitstellung eines gereinigten Gases (6,7) durch zumindest teilweise Abtrennung unerwünschter Gasbestandteile vom beladenen Gas, darunter zumindest eines von Ammoniak, Kohlenstoffdioxid und Schwefelwasserstoff, zur Erhöhung zumindest des Methananteils im Rohgas (5) mittels mindestens eines, bevorzugt temperierten, zweiten Reaktors (3), optional unter Verwendung von Wasser, neutralen, sauer oder basisch wirkenden Bestandteilen, Aufschlämmungen oder Eluaten, bevorzugt zumindest Wasser.

2. Verfahren nach Anspruch 1, wobei das Rohgas (5) und das Fermentationsmedium (4) durch Fermentation in einem Fermenter (1) gewonnen werden und zur Bereitstellung aus dem Fermenter (1) entnommen werden.

3. Verfahren nach Anspruch 2, wobei das gereinigte Gas zumindest teilweise in den Fermenter (1) zurückgeführt wird.

4. Verfahren nach einem der Ansprüche 1 bis 3, weiterhin aufweisend die Schritte:
- zumindest teilweise Rückführung des gereinigten Gases (6) in den ersten Reaktor (2) und erneutes Strippen zur Bereitstellung eines beladenen Gases,
- erneute Abtrennung der unerwünschten Gasbestandteile des beladenen Gases zur Bereitstellung eines gereinigten Gases (6,7) mittels des mindestens einen zweiten Reaktors (3).

5. Verfahren nach einem der Ansprüche 1 bis 4, wobei bei der Verminderung der im Fermentationsmedium (4) enthaltenen Gase gleichzeitig Ammoniak und Kohlenstoffdioxid aus dem Fermentationsmedium (4) entzogen werden, und
wobei bei der Abtrennung unerwünschter Gasbestandteile aus dem beladenen Gas entsprechend aus zumindest Ammoniak und Kohlenstoffdioxid gebildete Salze wie Ammoniumsalze und Carbonate ausgefällt oder als eine wässrige Lösung entnommen werden.

6. Verfahren nach einem der Ansprüche 1 bis 5, wobei das Fermentationsmedium (4) nach der Verminderung der darin enthaltenen Gase anschließend einer Fest-Flüssig-Phasentrennung (8) unterzogen wird.

7. Verfahren nach einem der Ansprüche 1 bis 6, wobei das Fermentationsmedium (4) kontinuierlich zwischen dem Fermenter (1) und dem ersten Reaktor (2) ausgetauscht wird, wobei im ersten Reaktor (2) zumindest eines von Ammonium in Form von Ammoniak, Kohlenstoffdioxid, Schwefelwasserstoff oder Methan aus dem Fermentationsmedium (4) entzogen wird und wobei das Fermentationsmedium (4) anschließend zumindest teilweise wieder in den Fermenter (1) rückgeführt wird.

8. Verfahren nach einem der Ansprüche 1 bis 7, wobei das entgaste Fermentationsmedium (4) nach dem ersten Reaktor (2) in einen dritten Reaktor (9) überführt wird, in welchem das Fermentationsmedium (4) mit einem zweiten Gas in Kontakt gebracht wird, sodass Fermentations-Organismen im Fermentationsmedium (4) inaktiviert werden.

9. Verfahren nach Anspruch 8, wobei als zweites Gas Luft, Abluft aus Klär- oder Industrieprozessen oder Abgas aus Verbrennungsprozessen verwendet wird, wobei in dem Gas befindliche Schadstoffe zumindest teilweise durch das Fermentationsmedium (4) absorbiert bzw. aus dem Gas entfernt werden.

10. Verfahren nach einem der Ansprüche 1 bis 9, wobei in den ersten Reaktor (2) weiterhin Fällmittel oder sauer bzw. basisch wirkende Stoffe oder Lösungen zugegeben werden, um das Absorptions-/Desorptions-Gleichgewicht von im Fermentationsmedium (4) enthaltene Gasbestandteilen während des Strippens gezielt zu verändern und im Fermentationsmedium (4) gelöste Bestandteile, insbesondere Phosphate, gezielt auszufällen.

11. Verfahren nach einem der Ansprüchen 1 bis 10, wobei in den ersten Reaktor (2) weiterhin Brüdenkondensat eingeführt wird, das gleichzeitig mit dem Fermentationsmedium (4) von enthaltenen Gasen teilweise befreit wird, wobei zumindest ein Teil des Brüdenkondensats optional durch Trocknung (16) des von enthaltenen Gasen zumindest teilweise befreiten und teilweise von Wasser abgetrennten Fermentationsmediums (4) generiert wird.

12. Verfahren nach einem der Ansprüchen 1 bis 11, wobei nach der Verminderung der im Fermentationsmedium (4) enthaltenen Gase durch Strippen im ersten Reaktor (2) die im Fermentationsmedium (4) enthaltenen Gase durch Vakuumentgasung (12) weiter vermindert werden.

13. Verfahren nach einem der Ansprüchen 1 bis 12, wobei das Rohgas (5) und/oder das beladene Gas und/oder das gereinigte Gas (6) weiterhin einen Gasfilter (11), insbesondere einen Kies- oder einen Aktivkohlefilter, zur weiteren Reinigung durchströmt.

14. Verfahren nach einem der Ansprüche 1 bis 13, wobei Wärmeenergie aus dem Verfahren rückgewonnen und bevorzugt zur Vorwärmung des Fermentationsmediums (4) vor dem ersten Reaktor (2) oder während eines Fermentationsprozesses genutzt wird.

15. Verfahren nach einem der Ansprüche 1 bis 14, wobei durch die Zugabe von Calciumsulfat im zweiten Reaktor Ammoniumsulfat und Calciumcarbonat gebildet werden.

16. Verfahren nach einem der Ansprüche 1 bis 15, wobei das gereinigte Gas (7) einer weitergehenden Nachbehandlung unterzogen wird, um einer energetischen oder stofflichen Nutzung zugeführt zu werden.

17. Verfahren nach Anspruch 5, wobei die Salze anschließend wieder gespalten werden, um Ammoniak und Kohlenstoffdioxid bereitzustellen.

18. Verfahren nach Anspruch 5, wobei die Salze anschließend chemisch mittels einer sauer wirkenden Substanz gespalten werden, um ein weiteres ammoniakhaltige Salz und Kohlenstoffdioxid in Gasform zu erhalten.

19. Verfahren nach Anspruch 5, wobei die Salze anschließend chemisch mittels einer basisch wirkenden Substanz gespalten werden, um ein weiteres Kohlendioxid enthaltendes Salz und Ammoniak in Gasform zu erhalten.
